(19) Europäisches Patentamt
European Patent Office
Office européen des brevets

(11) Numéro de publication: **0 080 401 B1**

(12) **FASCICULE DE BREVET EUROPEEN**

(45) Date de publication de fascicule du brevet: **29.07.92**

(51) Int. Cl.$^5$: **A61K 39/44**

(21) Numéro de dépôt: **82402078.8**

(22) Date de dépôt: **15.11.82**

Le dossier contient des informations techniques présentées postérieurement au dépôt de la demande et ne figurant pas dans le présent fascicule.

(54) **Nouveaux médicaments anticancéreux pour le traitement des leucémies T constitués de la chaine A de la ricine et d'un anticorps monoclonal spécifique.**

(30) Priorité: **20.11.81 FR 8121836**

(43) Date de publication de la demande:
**01.06.83 Bulletin 83/22**

(45) Mention de la délivrance du brevet:
**29.07.92 Bulletin 92/31**

(84) Etats contractants désignés:
**AT BE CH DE FR GB IT LI LU NL SE**

(56) Documents cités:
**EP-A- 0 023 401**
**EP-A- 0 044 167**
**FR-A- 2 437 213**
**FR-A- 2 466 252**

**NATURE, vol. 294, no. 5837, 12 novembre 1981, pages 171-173, Macmillan Journals, Chesham, Bucks., (GB);**

**NATURE, vol. 290, 12 mars 1981, pages 145-146; H.E. BLYTHMAN et al.**

(73) Titulaire: **ELF SANOFI**
**32-34, rue Marbeuf**
**F-75008 Paris(FR)**

(72) Inventeur: **Casellas, Pierre Résidence Le Parc des Arceaux**
**Bâtiment G5 rue Paul Rimbaud**
**F-34100 Montpellier(FR)**
Inventeur: **Gros, Pierre**
**18 rue des Muriers**
**F-34100 Montpellier(FR)**
Inventeur: **Jansen, Franz**
**Chemin des Sesquets**
**F-34160 Assas(FR)**

(74) Mandataire: **Gillard, Marie-Louise**
**Cabinet Beau de Loménie 55, Rue d'Amsterdam**
**F-75008 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

**CHEMICAL ABSTRACTS, vol. 95, 1981, page 24, no. 214896g, Columbus Ohio (USA); L.L.HOUSTON et al.**

**CHEMICAL ABSTRACTS, vol. 94, 1981, page 41, no. 132084f, Columbus Ohio (USA); B. PAU et al.**

**BIOLOGICAL ABSTRACTS, vol. 71, no. 7, 1981, no. 46810, PHiladelphia (USA); D.G. GILLILAND et al.**

**BIOLOGICAL ABSTRACTS, vol. 71, 1981, no. 32655, Philadelphia (USA);K.A.C. KROLIC et al.**

**CHEMICAL ABSTRACTS, vol. 96, 1982, page 584, no. 140919p, Columbus Ohio (USA); F.K. JANSEN et al.**

**CHEMICAL ABSTRACTS, vol. 96, 1982, page 465, no. 32919e, Columbus Ohio (USA); F.K. JANSEN et al.**

**CHEMICAL ABSTRACTS, vol. 95, 1981, page 57, no. 126185u, Columbus Ohio (USA); F.K. JANSEN et al.**

**CHEMICAL ABSTRACTS, vol. 95, 1981, page 568, no. 78306k, Columbus Ohio (USA); T.N. OELTMANN et al.**

**CHEMICAL ABSTRACTS, vol. 93, 1980, page 197, no. 232191m, Columbus Ohio (USA); R.J. YOULE et al.**

## Description

Dans ces brevets francais n° 2.437.213 et 2.466.252, la demanderesse a décrit la préparation de produits anti-cancéreux dits conjugués obtenus par couplage par liaison covalente de la chaîne A de la ricine à une structure protéique telle qu'un anticorps, une immunoglobuline ou un fragment d'immunoglobuline capable de reconnaître sélectivement un antigène donné à la surface des cellules porteuses que l'on veut atteindre telles que les cellules cancéreuses. La propriété principale de ces conjugués est d'être des agents cytotoxiques spécifiques des cellules cibles visées.

D'autre part, dans les documents ci-après on décrit des immunotoxines obtenues à partir d'anticorps ou de fragments d'anticorps issus d'espèces animales : rat et souris. Ces immunotoxines ne sont pas susceptibles d'être utilisées chez l'homme:

- NATURE, volume 290, 12 Mars 1981, pages 145-146 ;
- CHEMICAL ABSTRACTS, volume 95, 1981, numéro 21489g, Columbus Ohio (USA);
- CHEMICAL ABSTRACTS, volume 94, 1981, page 41, numéro 132084f, Columbus Ohio (USA);
- BIOLOGICAL ABSTRACTS, volume 71, numéro 7, 1981, numéro 46810, Philadelphia (USA);
- CHEMICAL ABSTRACTS, volume 93, 1980, page 197, numéro 232191 m, Columbus Ohio (USA);
- EP-A-0 023.401;
- BIOLOGICAL ABSTRACTS, volume 71, 1981, numéro 32655, Philadelphia (USA);
- CHEMICAL ABSTRACTS, volume 95, 1981, page 568, numéro 78306k, Columbus, Ohio (USA).

TROWBRIDGE et al. dans NATURE, volume 294, numéro 5837, 12 Novembre 1981, pages 171-173, Macmillan Journals, Chesham, Bucks., (GB), décrivent des immunotoxines obtenues à partir d'un anticorps dirigé contre les récepteurs de la transferrine. Ces récepteurs sont particulièrement nombraux sur certaines cellules tumorales, mais ils existent aussi sur d'autres cellules à prolifération rapide et notamment sur les progéniteurs érythroïdes. Les immunotoxines décrites dans ce document ne peuvent donc pas être utilisées en thérapeutique humaine.

L'utilisation d'anticorps dirigés contre les antigènes de différentiation des cellules cancéreuses avait déjà permis d'obtenir des conjugués présentant une spécificité notable vis-à-vis des cellules cibles.

La présente invention a pour objet à titre de médicaments des produits dits conjugés obtenus à partir de la chaîne A de la ricine et d'un anticorps monoclonal anti-cellules T humaines.

On entendu par conjugués des molécules mixtes artificielles dans lesquelles la chaîne A de la ricine est associée à une liaison covalente de type disulfure à un anticoprs anti-cellules T humaines capable de reconnaître sélectivement un antigène associé à des cellules cancéreuses.

L'obtention de la chaîne A de ricine pure a été décrite dans nos brevets antérieurs. La préparation d'anticoprs monoclonaux dirigés contre des cellules leucémiques T humaines a été mentionnée dans la littérature scientifique. (On peut se reporter en particulier à Journal of Immunology 125 (2), 725-737, (1980).

Pour réaliser les conjugués, il est nécessaire que les protéines à coupler portent chacune au moins un atome de soufre naturellement apte ou artificiellement rendu apte à créer la liaison disulfure recherchée.

La chaîne A de ricine présente naturellement un seul atome de soufre permettant le couplage souhaité. C'est celui de la fonction thiol du résidu de cystéine inclus dans la chaîne A et qui assurait la liaison de cette chaîne A à la chaîne B dans la toxine complète.

L'anticorps entier de spécificité anti-cellules T humaines ne comporte ni fonction thiol libre ni d'autres atomes de soufre susceptibles d'être utilisés pour le couplage. Si l'on utilise cet anticorps entier, il conviendra donc d'introduire artificiellement sur la molécule d'immunoglobuline un ou plusieurs atomes de soufre susceptibles d'être engagés ultérieurement dans la liaison disulfure à établir avec une ou plusieurs molécules de chaîne A de ricine.

Si par contre on utilise le fragment Fab' de cet anticorps tel qu'il est classiquement obtenu par protéolyse limitée en présence de pepsine suivie d'une réduction du (ou des) ponts disulfure entre chaînes lourdes, ce fragment présente alors au moins un groupement thiol disponible pour créer le pont disulfure avec la chaîne A de ricine, Dans ce dernier cas, selon l'invention, le groupement thiol disponible sur le fragment d'anticorps sera en général transformé par des méthodes connues en disulfure mixte activé, avant d'être mis en réaction avec le thiol de la chaîne A pour créer le groupement disulfure recherché.

Si l'on utilise l'anticorps entier selon l'invention, le préparation du conjugué est effectuée en mettant en présence la chaîne A de ricine porteuse de son groupement SH libre avec l'anticorps dans lequel le groupement SH a été artificiellement introduit sous forme activée et notamment sous la forme d'un disulfure mixte avec un radical organique soufré convenable.

La préparation du conjugué peut alors être représentée par le schéma :

$$RA - SH + AC - R - S - S - X \rightarrow RA - S - S - R - AC + XSH$$

dans lequel :

- RA désigne la chaîne A de ricine
- AC désigne l'anticorps
- X désigne le radical activateur.

L'anticorps substitué par un atome de soufre activé est obtenu a partir de l'anticorps lui-même, par substitution à l'aide d'un réactif (lui-même porteur d'un atome de soufre activé selon le schéma :

AC + Y - R - S - S - X → AC - R - S - S - X

dans lequel :

- AC désigne l'anticorps
- Y représente une fonction permettant la fixation covalente du réactif sur la protéine
- R désigne un groupement pouvant porter simultanément les substituants AC et - S - S - X
- X désigne le radical activateur.

Le groupement fonctionnel Y est une fonction capable de se lier de façon covalente avec l'une quelconque des fonctions portées par les chaînes latérales des aminoacides constitutifs de la protéine à substituer. Parmi celles-ci, les fonctions aminées terminales des radicaux lysyle contenues dans la protéine sont particulièrement indiquées. Dans ce cas, Y pourra notamment représenter :

- un groupe carboxylique qui pourra se lier aux fonctions aminées de la protéine en présence d'un agent de couplage tel qu'un carbodiimide et notamment un dérivé soluble dans l'eau comme l'éthyl-l (diéthylamino-3 propyl)-3 carbodiimide,
- un chlorure d'acide carboxylique qui est susceptible de réagir directement avec les fonctions aminées pour les acyler,
- un ester dit "activé" tel qu'un ester d'ortho- ou para-, nitro- ou dinitro-phényle ou encore un ester de N-hydroxy succinimide qui réagit directement avec les fonctions aminées pour les acyler,
- un anhydride interne d'un diacide carboxylique tel, par exemple, l'anhydride succinique qui réagit spontanément avec les fonctions amines pour créer des liaisons amides,
- un groupement imidoester

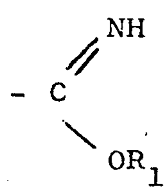

où $R_1$

est un groupe alkyle réagissant avec les groupes aminés de la protéine selon la réaction :

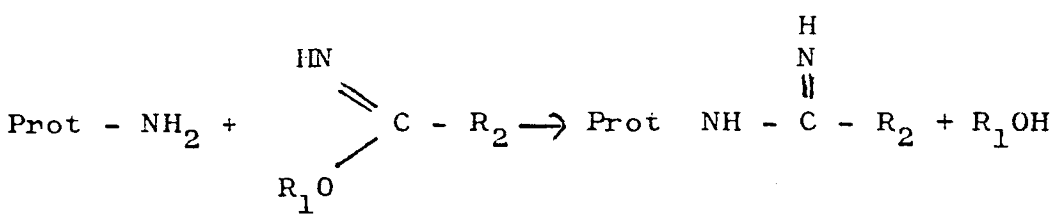

Le radical -S-S-X désigne un disulfure mixte activé capable de réagir avec un radical thiol libre. En particulier dans ce disulfure mixte, X pourra désigner un groupe pyridyl-2 ou pyridyl-4 éventuellement substitué par un ou des radicaux alkyle, halogène, carboxyliques. X peut aussi désigner un groupe phényle de préférence substitué par un ou des groupes nitro- ou carboxyliques. X peut encore représenter un groupe alcoxycarbonyle tel que le groupe méthoxycarbonyle.

Le radical R désigne tout radical capable de porter simultanément les substituants AC et S - S - X. Il devra être choisi de façon à ne pas comporter de fonctions susceptibles d'interférer au cours des réactions ultérieures avec les réactifs utilisés et les produits synthétisés. En particulier, le groupe R peut être un groupe - $(CH_2)_n$ avec n compris entre 1 et 10, ou encore un groupe :

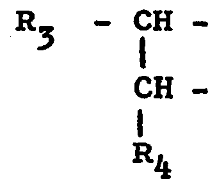

dans lequel $R_4$ désigne l'hydrogène ou un groupe alkyle ayant de 1 à 8 atomes de carbone et $R_3$ désigne un substituant inerte vis-à-vis des réactifs utilisés ultérieurement tel qu'un groupe carbamate

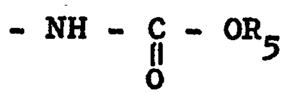 où $R_5$

désigne un groupe alkyle droit ou ramifié ayant de 1 à 5 atomes de carbone et notamment le groupe tertiobutyle.

La réaction du composé Y - R - S - S - X avec l'immunoglobuline est effectuée en phase liquide homogène le plus souvent dans l'eau ou une solution tampon. Lorsque la solubilité des réactifs l'exige, il est possible d'ajouter au milieu réactionnel jusqu'à 20% en volume d'un solvant organique miscible à l'eau tel qu'un alcool et notamment le butanol tertiaire.

La réaction est effectuée à température ambiante pendant un temps variant de quelques heures à 24 heures. Après quoi, une dialyse permet d'éliminer les produits de faible masse moléculaire et, en particulier, les excès de réactifs. Ce procédé permet d'introduire un nombre de groupements substituants par mole de protéine compris entre 1 et 5 si la protéine est une immunoglobuline de classe G, compris entre 1 et 15 si la protéine est une immunoglobuline de classe M.

En utilisant de tels composés, le couplage avec la chaîne A de ricine est réalisé par mise en présence en solution aqueuse des deux protéines à une température ne dépassant pas 30°C pendant un temps variant de quelques heures à un jour. La solution obtenue est dialysée pour éliminer les produits de faible masse moléculaire, puis le conjugué peut être purifié par diverses méthodes connues.

L'exemple suivant permet de mieux comprendre l'invention sans en limiter la portée.

EXEMPLE 1 :

Conjugué obtenu par réaction entre un anticorps anti-cellules T humaines (anticorps dirigé contre l'antigène T65) substitué par un groupe disulfure activé et la chaîne A de la ricine.

a) Anticorps anti-cellules T humaines (ou anticorps T101)

Cet anticorps a été obtenu selon la méthode décrite dans Journal of Immunology 125 (2), 725-737 (1980).

Il subit une ultime purification par dislyse contre du tampon PBS (10 mM de phosphate, 140 mM de chlorure de sodium, pH 7,4).

b) Chaîne A de la ricine :

La chaîne A de la ricine a été préparée et purifiée ainsi qu'il a été indiqué dans nos demandes antérieures (brevet No. 78/27838 et addition No. 79/24655).

c) Anticorps anti-cellules T humaines activé :

A 0,5 ml d'une solution 14,2 mg/ml d'acide (pyridyl-2 disulfanyl)-3 propionique dans le tertiobutanol, on ajoute 0,1 ml d'une solution à 42,7 mg/ml d'éthyl-1 (dimethylamino-3 propyl)-3 carbodiimide et laisse 3 minutes à température ambiante.

On ajoute 180 µl de la solution ainsi obtenue à 5,6 ml d'une solution d'anticorps à 3,6 mg/ml dans le tampon PBS. On laisse l'incubation se poursuivre pendant 20 heures à 30°C.

On dialyse ensuite la solution en continue pendant 3 jours contre 21 litres de tampon PBS à 4°C. On obtient ainsi 16 mg d'anticorps activé à une concentration de 2,6 mg/ml.

Par dosage spectrophotométrique à 343 nm de la pyridine thione-2 libérée par échange avec le glutathion réduit, on constate que l'on a obtenu un anti-un anticorps portant 3,1 groupements activateurs par mole d'anticorps.

d) Conjugué :

A 4,6 ml d'une solution d'anticorps activé dans le tampon PBS (concentration 2,6 mg/ml, soit 12 mg

d'anticorps activé), on ajoute 0,87 ml d'une solution de chaîne A de ricine dans le même tampon (concentration 6,6 mg/ml) et effectue l'incubation à 25° C pendant 20 heures.

On chromatographie le mélange réactionnel sur colonne de gel de "Sephadex G100"®.Dans chaque fraction, on détermine la concentration en anticorps par spectrophotométrie à 280 nm et celle de la chaîne A par son pouvoir inhibiteur de la protéosynthèse mesurée sur un système acellulaire. On réunit les fractions identiques contenant le conjugué et on obtient ainsi environ 11 mg du conjugué à la concentration de 0,8 mg/ml.

Les déterminations analytiques effectuées permettent de montrer que la solution contient 140 µg/ml de chaîne A biologiquement active, soit environ 1,1 mole de chaîne A par mole d'anticorps.

Une étude effectuée par cytofluorométrie a en outre permis de montrer que l'anticorps anti-cellules T humaines utilisé, l'anticorps activé correspondant et le conjugué de cet anticorps avec la chaîne A de la ricine présentent des histogrammes de fluorescence superposables permettant d'affirmer que l'anticorps n'a subi aucune altération importante aux cours des réactions d'activation et de couplage auxquelles il a été soumis et en particulier qu'il reste capable, au sein même du conjugué, de reconnaître l'antigène T humain contre lequel il est dirigé.

Le conjugué, selon l'invention, obtenu précédemment a été étudié en ce qui concerne ses propriétés biologiques et plus spécialement son action anti-cancéreuse.

## 1) - INHIBITION DE LA PROTEOSYNTHESE

La propriété biologique fondamentale de la chaîne A de ricine est d'inhiber la protéosynthèse cellulaire par altération de la sous-unité ribosomale 60 S.

On a utilisé ici un modèle cellulaire. Ce test mesure l'effet des substances étudiées sur l'incorporation de $^{14}$C-leucine dans les cellules cancéreuses en culture.

Les cellules utilisées appartiennent à la lignée cellulaire CEM issue d'une leucémie T humaine qui porte l'antigène T65. Les cellules sont incubées en présence de la substance à étudier puis, en fin d'incubation, on procède à la mesure du taux d'incorporation de $^{14}$C-leucine par les cellules ainsi traitées.

Cette mesure est effectuée selon une technique adaptée de la technique décrite dans Journal of Biological Chemistry 1974, 249 (11), 3557-62 utilisant le traceur $^{14}$C-leucine pour la détermination du taux de protéosynthèse. La détermination de la radioactivité incorporée est effectuée ici sur les cellules entières isolées par filtration.

A partir de ces déterminations, on peut tracer les courbes effets/doses présentant en abscisse la concentration molaire en chaîne A des substances étudiées et en ordonnée l'incorporation de $^{14}$C-leucine exprimée en pourcentage de l'incorporation des cellules témoins en l'absence de toute substance affectant la synthèse protéique.

On peut ainsi déterminer pour chaque substance étudiée la concentration qui inhibe 50% de l'incorporation de $^{14}$C-leucine ou "concentration inhibitrice 50" (CI 50).

La figure 1 représente les courbes obtenues dans la même expérience avec la ricine, avec sa chaîne A libre et avec le conjugué RT2, (composé préparé selon l'exemple 1), en présence et en l'absence de chlorure d'ammonium 10 mM dans le milieu d'incubation. Même l'absence de chlorure d'ammonium et après incubation de 48 h on peut constater sur cette figure que le conjugué étudié RT2 présente une forte activité cytotoxique (CI 50 = $7 \times 10^{-12}$M), environ 7000 fois supérieure à celle de la chaîne A de ricine.

## 2) - POTENTIALISATION DE L'ACTIVITE DU CONJUGUE RT2 PAR LE CHLORURE D'AMMONIUM

Comme le montre aussi la figure 1, la présence de chlorure d'ammonium 10 mM dans le milieu d'incubation des cellules avec le conjugué RT2, augmente de façon très importante - environ 80 fois - l'activité cytotoxique du conjugué sur les cellules-cibles. Cet effet potentialisateur n'est pas obtenu ni avec la ricine, ni avec la chaîne A, ni avec un conjugué non spécifique des cellules étudiées. Ainsi, en présence de chlorure d'ammonium comme agent potentialisateur, l'activité cytotoxique du conjugué (CI 50 = $8{,}5.10^{-14}$ M) devient environ 600 000 fois plus élevée que celle de la chaîne A seule et dépasse même en puissance l'activité de la ricine, ce qui n'a jamais été décrit pour aucun conjugué entre la chaîne A de ricine et un anticorps quelconque.

Les conjugués préparés selon l'invention présentent par eux-mêmes une forte activité cytotoxique spécifique vis-à-vis des lignées cellulaires de leucémies T humaines. Ils peuvent donc être utilisés en thérapeutique humaine dans le traitement des leucémies T ou de toute autre affection,cancéreuse ou non, dans laquelle il conviendrait de détruire sélectivement des cellules qui seraient sensibles au conjugué préparé selon l'invention. Ceci est envisageable dans les transplantations ou dans certaines maladies autoimmunes pour diminuer l'activité des lymphocytes T ou de telle sous-population de lymphocytes T.

Si l'on tient compte en outre de l'effet potentialisateur très important du chlorure d'ammonium sur l'activité de ces conjugués vis-à-vis des cellules-cibles correspondantes, il est possible de tirer parti de cette propriété nouvelle en vue d'accroître l'efficacité thérapeutique du conjugué dans le traitement des maladies auxquelles il s'applique. Cette ammélioration peut être exploitée selon deux schémas thérapeutiques différents :

a) on peut traiter in vitro par le conjugué RT2 en présence de chlorure d'ammonium des prélèvements de moëlle osseuse humaine provenant de patients notamment leucémiques. Cette association présentant une efficacité cytotoxique très élevée ettrès spécifique, il est possible d'éliminer de la moëlle ainsi traitée toute cellule portant l'antigène reconnu par le conjugué et en particulier toute cellule tumorale. Par ailleurs, le patient peut être traité par toute thérapeutique appropriée telle que radiothérapie et/ou chimiothérapie à dose supralétale de façon à éliminer les cellules tumorales de son organisme. Enfin la moëlle, purifiée comme indiqué, est transplantée à nouveau dans l'organisme du patient sous forme de greffe autologue pour permettre la reconstitution des populations de cellules sanguines détruites par le traitement supralétale.

b) l'application de la propriété potentialisatrice du chlorure d'ammonium chez le patient in vivo peut aussi être exploitée pour obtenir l'efficacité maximale du conjugué. En effet, il a été montré que des souris chez qui on a préalablement transplanté des cellules tumorales et à qui on administre le conjugué en même temps que 3 injections de 7 mg de chlorure d'ammonium espacées toutes les 15 minutes, présentent un meilleur pourcentage de survie et une meilleure inhibition de croissance de la tumeur que ne présentent les souris-témoins ayant reçu le conjugué seul.

Ces conjugués sont conditionnés pour être utilisés par voie injectable. Ils peuvent être utilisés soit seuls, soit associés au chlorure d'ammonium, soit associés à un autre traitement de l'affection cancéreuse concernée et, en particulier, associés à d'autres médicaments immunodépresseurs afin de retarder et d'affaiblir la réaction immunitaire naturelle du patient vis-à-vis de la protéine étrangère à son organisme que représente le conjugué.

Visant à éliminer la totalité des cellules cancéreuses, le traitement devra être effectué avec une dose suffisante de conjugué et la durée du traitement devra être déterminée dans chaque cas en fonction du sujet et de la nature de l'affectation à traiter.

**Revendications**

**Revendications pour les Etats contractants suivants : DE, GB, FR, IT, NL, SE, BE, LU, CH, LI**

1. Médicament anticancéreux pour l'homme, caractérisé en ce qu'il contient au moins un conjugué obtenu par couplage, au moyen d'un pont disulfure, de la chaîne A de la ricine avec un anticorps monoclonal anticellules T humaines, ou un fragment d'un tel anticorps, ledit conjugué étant un agent cytotoxique spécifique desdites cellules T.

2. Médicament anticancéreux selon la revendication 1, caractérisé en ce que ledit anticorps monoclonal est un anticorps monoclonal entier sur lequel a été introduit le groupement SH activé.

3. Médicament selon la revendication 2, caractérisé en ce que ledit groupement SH activé est sous la forme d'un pont disulfure reliant ledit anticorps et un radical organique activateur connu.

4. Médicament selon la revendication 1, caractérisé en ce que le fragment dudit anticorps est le fragment Fab' de celui-ci.

5. Médicament selon l'une quelconque des revendications 1 à 4, caractérisé en ce qu'il contient en outre du chlorure d'ammonium.

6. Médicament selon l'une quelconque des revendications 1 à 5, caractérisé en ce que l'anticorps anticellules T humaines est l'anticorps T101.

**7.** Procédé de préparation du principe actif du médicament selon l'une quelconque des revendications 1 à 6, caractérisé en ce que :
- l'on utilise un anticorps substitué par un atome de soufre activé ayant pour formule :

  AC - R - S - S - X

  dans laquelle AC est l'anticorps monoclonal choisi, S est le soufre, X désigne un radical activateur et R est un groupe pouvant porter simultanément les substituants AC et - S - S - X ;
- et que l'on fait réagir ledit produit sur le thiol de la chaîne A de la ricine.

**8.** Procédé de préparation du principe actif du médicament selon la revendication 4, caractérisé en ce que l'on prépare tout d'abord, selon des procédés connus, le fragment Fab' de l'anticorps, puis on transforme ledit fragment en un disulfure mixte activé que l'on fait réagir avec le thiol de la chaîne A de la ricine.

**9.** Utilisation du chlorure d'ammonium pour la préparation d'un agent potentialisateur de l'activité de conjugués constitués par la chaîne A de la ricine couplée par un pont disulfure avec un anticorps monoclonal anticellules T humaines, ou un fragment d'un tel anticorps, ledit conjugué étant un agent cytotoxique spécifique desdites cellules T.

**Revendications pour l'Etat contractant suivant : AT**

**1.** Procédé pour l'obtention d'un médicament anticancéreux pour l'homme, caractérisé en ce qu'il consiste à utiliser au moins un conjugué obtenu par couplage, au moyen d'un pont disulfure, de la chaîne A de la ricine avec un anti corps monoclonal anticellules T humaines, ou un fragment d'un tel anticorps, ledit conjugué étant un agent cytotoxique spécifique desdites cellules T.

**2.** Procédé selon la revendication 1, caractérisé en ce que ledit anticorps monoclonal est un anticorps monoclonal entier sur lequel a été introduit le groupement SH activé.

**3.** Procédé selon la revendication 2, caractérisé en ce que ledit groupement SH activé est sous la forme d'un pont disulfure reliant ledit anticorps et un radical organique activateur connu.

**4.** Procédé selon la revendication 1, caractérisé en ce que le fragment dudit anticorps monoclonal est le fragment Fab' de celui-ci.

**5.** Procédé selon l'une quelconque des revendications 1 à 4, caractérisé en ce que l'on ajoute en outre du chlorure d'ammonium.

**6.** Procédé selon l'une quelconque des revendications 1 à 6, caractérisé en ce que l'on utilise l'anticorps T101 comme anticorps anticellules T humaines.

**7.** Procédé selon l'une quelconque des revendications 1 à 6, caractérisé en ce que :
- l'on utilise un anticorps substitué par un atome de soufre activé ayant pour formule :

  AC - R - S - S - X

  dans laquelle AC est l'anticorps monoclonal choisi, S est le soufre, X désigne un radical activateur et R est un groupe activateur pouvant porter simultanément les substituants AC et - S - S - X ;
- et que l'on fait réagir ledit produit sur le thiol de la chaîne A de la ricine.

**8.** Procédé selon la revendication 4, caractérisé en ce que l'on prépare tout d'abord, selon des procédés connus, le fragment Fab' de l'anticorps, puis on transforme ledit fragment en un disulfure mixte activé que l'on fait réagir avec le thiol de la chaîne A de la ricine.

**9.** Utilisation du chlorure d'ammonium pour la préparation d'un agent potentialisateur de l'activité de conjugués constitués par la chaîne A de la ricine couplée par un pont disulfure avec un anticorps

monoclonal anticellules T humaines, ou un fragment d'un tel anticorps, ledit conjugué étant un agent cytotoxique spécifique desdites cellules T.

**Claims**

**Claims for the following Contracting States : DE, GB, FR, IT, NL, SE, BE, LU, CH, LI**

1. Anti-cancer drug for human, characterized in that it contains at least one conjugate obtained by coupling, by means of a disulphide bridge, of the A chain of the ricin with an anti-human T cell monoclonal antibody or with a fragment of such an antibody, said conjugate being a cytotoxic agent specific for said T cells.

2. Drug according to claim 1, characterized in that said monoclonal antibody is a whole antibody on which the activated SH group has been introduced.

3. Drug according to claim 2, characterized in that said activated SH group is under the form of a disulphide bridge bonding said antibody and a known organic activator radical.

4. Drug according to claim 1, characterized in that the fragment of said antibody is a Fab' fragment thereof.

5. Drug according to anyone of claims 1 to 4, characterized in that it further contains ammonium chloride.

6. Drug according to anyone of claims 1 to 5, characterized in that the anti-human T cell antibody is antibody T 101.

7. Process for the preparation of the active principle of the drug according to anyone of claims 1 to 6, characterized in that:
   - an antibody substituted by an activated sulfur atom having the formula:

     AC - R - S - S - X

     in which AC is the selected antibody, S is the sulfur atom, X is an activator radical and R is a group being capable to simultaneously bear the substituents AC and -S-S-X is used
   - and in that said compound is reacted on the thiol of the A chain of the ricin.

8. Process for the preparation of the active principle of the drug according to claim 4, characterized in that the Fab' fragment of the antibody is firstly prepared according to known methods and is then converted into an activated mixed disulphide which is reacted with the thiol of the A chain of the ricin.

9. Use of the ammonium chloride for the preparation of a potentiator agent for the activity of the conjugates constituted by the A chain of the ricin, coupled by a disulphide bridge with a monoclonal anti-human cell T antibody or a fragment of such an antibody, said conjugates being a cytotoxic agent specific for said T cells.

**Claims for the following Contracting State : AT**

1. Process for the obtention of an anti cancer drug for human, characterized in that it consists in using at least one conjugate obtained by coupling, by means of a disulphide bridge, of the A chain of the ricin with an anti-human T cell monoclonal antibody or with a fragment of such an antibody, said conjugate being a cytotoxic agent specific for said T cells.

2. Process according to claim 1, characterized in that said monoclonal antibody is a whole antibody on which the activated SH group has been introduced.

3. Process according to claim 2, characterized in that said activated SH group is under the form of a disulphide bridge bonding said antibody and a known organic activator radical.

4. Process according to claim 1, characterized in that the fragment of said monoclonal antibody is the

fragment Fab' thereof.

5. Process according to anyone of claims 1 to 4, characterized in that ammonium chloride is further added.

6. Process according to anyone of claims 1 to 5, characterized in that antibody T 101 is used as anti-human T cell antibody.

7. Process according to anyone of claims 1 to 6, characterized in that:
   - an antibody substituted by an activated sulfur atom having the formula:

     AC - R - S - S - X

     in which AC is the selected antibody, S is the sulfur atom, X is an activator radical and R is a group being capable to simultaneously bear the substituents AC and -S-S-X is used
   - and in that said compound is reacted on the thiol of the A chain of the ricin.

8. Process for the preparation of the active principle of the drug according to claim 4, characterized in that the Fab' fragment of the antibody is firstly prepared according to known methods and is then converted into an activated mixed disulphide which is reacted with the thiol of the A chain of the ricin.

9. Use of the ammonium chloride for the preparation of a potentiator agent for the activity of the conjugates constituted by the A chain of the ricin, coupled by a disulphide bridge with a monoclonal anti-human cell T antibody or a fragment of such an antibody, said conjugates being a cytotoxic agent specific for said T cells.

**Patentansprüche**
**Patentansprüche für folgende Vertragsstaaten : DE, GB, FR, IT, NL, SE, BE, LU, CH, LI**

1. Antikrebs-Heilmittel für den Menschen, dadurch gekennzeichnet, dass es mindestens ein durch Kopplung, mittels einer Disulfid-Brücke, der Kette A des Ricins mit einem monoklonalen menschlichen Anti-Zellen T Antikörper oder einem Fragment eines solchen Antikörpers erhaltenes Konjugat enthält, wobei das genannte Konjugat ein spezifischer zytotoxischer Wirkstoff der genannten Zellen T ist.

2. Antikrebs-Heilmittel nach Anspruch 1, dadurch gekennzeichnet, dass der genannte monoklonale Antikörper ein ganzer monoklonaler Antikörper ist, auf dem die aktivierte SH Gruppierung eingeführt wurde.

3. Heilmittel nach Anspruch 2, dadurch gekennzeichnet, dass die genannte aktivierte SH Gruppierung in Form einer Disulfid-Brücke ist, die den Antikörper und ein bekanntes organisches Aktivatorradikal verbindet.

4. Heilmittel nach Anspruch 1, dadurch gekennzeichnet, dass das Fragment des genannten monoklonalen Antikörpers dessen Fragment Fab' ist.

5. Heilmittel nach einem der Ansprüche 1 bis 4, dadurch gekennzeichnet, dass man unter anderem Ammoniumchlorid hinzufügt.

6. Heilmittel nach einem der Ansprüche 1 bis 5, dadurch gekennzeichnet, dass der menschlichen Anti-Zellen T Antikörper der Antikörper T101 ist.

7. Verfahren zur Herstellung des aktiven Wirkstoffs des Heilmittels nach einem der Ansprüche 1 bis 6, dadurch gekennzeichnet, dass
   - man einen einen Antikörper verwendet, der durch ein aktiviertes Schwefelatom der Formel :

     AC - R - S - S - X

     substituiert ist, worin AC der gewählte monoklonale Antikörper ist, S Schwefel ist, X ein Aktivatorradikal bezeichnet und R eine Gruppe ist, die gleichzeitig die Substituenten AC und - S -

S - X tragen kann;
- und dass man das genannte Produkt mit Thiol der Kette A des Ricins reagieren lässt.

**8.** Verfahren zur Herstellung des aktiven Wirkstoffs des Heilmittels nach Anspruch 4, dadurch gekennzeichnet, dass man zunächst nach bekannten Verfahren das Fragment Fab' des Antikörpers herstellt, dass man dann das genannte Fragment in aktiviertes Misch-Disulfid umwandelt, das man mit dem Thiol der Kette A des Ricins reagieren lässt.

**9.** Verwendung von Ammoniumchlorid zur Herstellung eines Wirkstoffs, der die Aktivität von Konjugaten potentialisiert, die durch die durch eine Disulfid-Brücke mit einem monoklonalen menschlichen Anti-Zellen T Antikörper, oder mit einem Fragment eines solchen Antikörpers, gekoppelte Kette A des Ricins gebildet werden, wobei das genannte Konjugat ein spezifischer zytotoxischer Wirkstoff der genannten Zellen T ist.

**Patentansprüche für folgenden Vertragsstaat : AT**

**1.** Verfahren zur Herstellung eines Antikrebs-Heilmittels für den Menschen, dadurch gekennzeichnet, dass es darin besteht, mindestens ein durch Kopplung, mittels einer Disulfid-Brücke,der Kette A des Ricins mit einem monoklonalen menschlichen Anti-Zellen T Antikörper oder einem Fragment eines solchen Antikörpers erhaltenes Konjugat zu verwenden, wobei das genannte Konjugat ein spezifischer zytotoxischer Wirkstoff der genannten Zellen T ist.

**2.** Verfahren nach Anspruch 1, dadurch gekennzeichnet, dass der genannte monoklonale Antikörper ein ganzer monoklonaler Antikörper ist, auf dem die aktivierte SH Gruppierung eingeführt wurde.

**3.** Verfahren nach Anspruch 2, dadurch gekennzeichnet, dass die genannte aktivierte SH Gruppierung in Form einer Disulfid-Brücke ist, die den Antikörper und ein bekanntes organisches Aktivatorradikal verbindet.

**4.** Verfahren nach Anspruch 1, dadurch gekennzeichnet, dass das Fragment des genannten monoklonalen Antikörpers dessen Fragment Fab' ist.

**5.** Verfahren nach einem der Ansprüche 1 bis 4, dadurch gekennzeichnet, dass man unter anderem Ammoniumchlorid hinzufügt.

**6.** Verfahren nach einem der Ansprüche 1 bis 5, dadurch gekennzeichnet, dass man den Antikörper T101 als menschlichen Anti-Zellen T Antikörper verwendet.

**7.** Verfahren nach einem der Ansprüche 1 bis 6, dadurch gekennzeichnet, dass :
- man einen einen Antikörper verwendet, der durch ein aktiviertes Schwefelatom der Formel :

  AC - R - S - S - X

  substituiert ist, worin AC der gewählte monoklonale Antikörper ist, S Schwefel ist, X ein Aktivatorradikal bezeichnet und R eine Aktivator-Gruppe ist, die gleichzeitig die Substituenten AC und - S - S - X tragen kann;
- und dass man das genannte Produkt mit Thiol der Kette A des Ricins reagieren lässt.

**8.** Verfahren nach Anspruch 4, dadurch gekennzeichnet, dass man zunächst nach bekannten Verfahren das Fragment Fab' des Antikörpers herstellt, dass man dann das genannte Fragment in aktiviertes Misch-Disulfid umwandelt, das man mit dem Thiol der Kette A des Ricins reagieren lässt.

**9.** Verwendung von Ammoniumchlorid zur Herstellung eines Wirkstoffs, der die Aktivität von Konjugaten potentialisiert, die durch die durch eine Disulfid-Brücke mit einem monoklonalen menschlichen Anti-Zellen T Antikörper, oder mit einem Fragment eines solchen Antikörpers, gekoppelte Kette A des Ricins gebildet werden, wobei das genannte Konjugat ein spezifischer zytotoxischer Wirkstoff der genannten Zellen T ist.

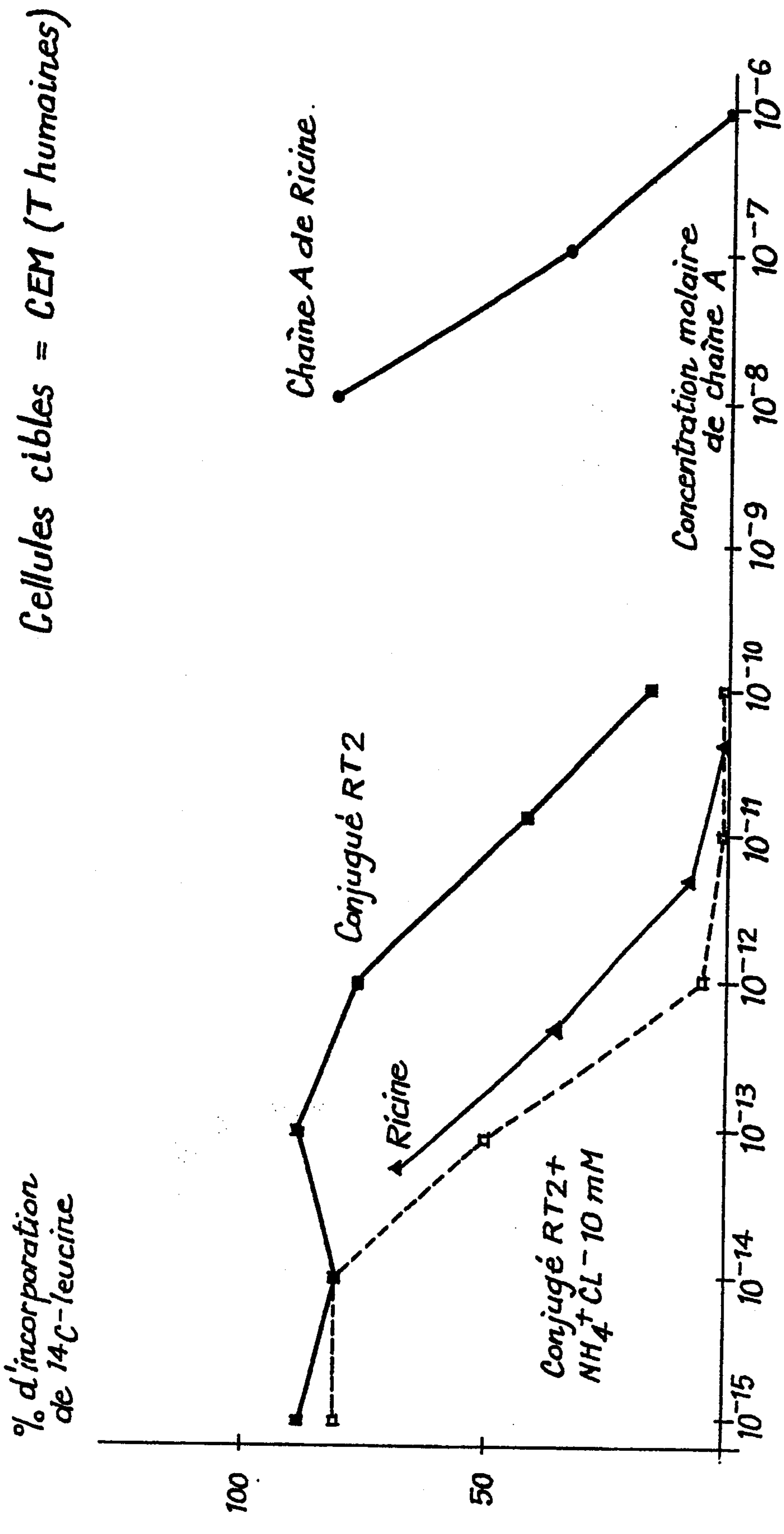
Cellules cibles = CEM (T humaines)
% d'incorporation de 14C-leucine
100
50
Chaîne A de Ricine
Conjugué RT2
Ricine
Conjugué RT2 + NH4+Cl- 10 mM
Concentration molaire de chaîne A
10-15
10-14
10-13
10-12
10-11
10-10
10-9
10-8
10-7
10-6